# EUROPEAN PATENT APPLICATION

(11) **EP 1 849 497 A1**
(43) Date of publication of application: **31.10.2007**
(21) Application number: 06113124.9
(22) Date of filing: 26.04.2006
(51) Int. Cl.: A61N 5/06

(54) **Tanning apparatus**

(71) Applicant: Koninklijke Philips Electronics N.V., 5621 BA Eindhoven (NL)
(72) Inventor: The designation of the inventor has not yet been filed
(74) Representative: Damen, Daniel Martijn

(57) **Abstract**

Indoor tanning, especially in commercial establishments which provide tanning beds, has been a very popular endeavour for the last few decades, particularly in view of concerns for exposure to natural sunlight and ultra-violet (UV) light. The invention relates to an improved tanning apparatus, having multiple light sources (6a, 6b), filtering means (8a, 8b) and reflecting means (9a, 9b).

## Description

The invention relates to a tanning apparatus.

Indoor tanning, especially in commercial establishments which provide tanning beds, has been a very popular endeavour for the last few decades. However, there are concerns for exposure to natural sunlight and ultra-violet (UV) light. It has been shown a link between UV exposure and skin cancer.

The most common method of skin tanning involves the process of exposing skin to UV radiation. Health research has shown that the condition of over-exposure to UV radiation causes a variety of health problems. Health care professionals believe that UV radiation can alter immune system functions. When UV radiation suppresses immune responses, the body's ability to fight certain diseases, including skin cancer, is reduced. It is suspected that over-exposure to UV radiation also interferes with the effectiveness of immunizations given through the skin. To take appropriate measures against (over)exposure to UV radiation during tanning, there is a need to modify the conventional, relatively unhealthy tanning process.

It is an object of the invention to provide an improved tanning apparatus for relatively healthy treatment, in particular tanning, of the skin.

This object can be achieved by providing an apparatus according to the preamble, comprising: multiple light sources with a spectral emittance concentrated in at least one specific narrow spectral band, wherein one spectral band is in the range of 400-440 nm, filtering means for at least substantially rejecting ultraviolet radiation emitted by said light sources, and reflecting means for directing light emitted by said light sources towards said filtering means. It has been found that violet/blue light emitted by light sources with a wavelength of between 400-440 nm is useful for tanning purposes. Although the spectral band of 400-440 nm is considered to be UV free, the tanning capacity of a such a light source as such is sufficient to achieve a satisfying tanning effect. Therefore, to achieve a satisfying tanning effect in an acceptable period of time (like e.g. 30 minutes), the application of both the reflecting means and multiple light sources are applied for increasing the energy density of the tanning apparatus according to the invention to achieve an intensivation and hence quickening of the (substantially) UV free tanning process. Therefore, the efficiency of the UV free tanning process can be improved significantly in this manner. Since light sources like high intensity discharge lamps, will also emit ultraviolet radiation during operation, the filtering means are applied to substantially filter out this UV radiation being harmful for the skin. Therefore, mere the combination of technical features will result in a satisfying tanning apparatus with which a user's skin can be tanned in a relatively healthy manner. It has been shown that the apparatus according to the invention is also suitable for treatment of acne.

Commonly, the light sources are enclosed at least partially by the reflecting means and the filtering means. In a preferred embodiment the tanning apparatus comprises at least one housing for at least one light source, in which housing the filtering means and the reflecting means are incorporated. In this manner light emitted by the light sources can be used in an optimum manner for the tanning process. More specifically, light emitted by the light source can be reflected in an optimum, commonly converging manner in the direction of the filtering means to irradiate a user in an efficient and effective manner. Said housing may be adapted for containing multiple light sources, wherein the mutual orientation of the light sources may vary. Preferably, in this latter case the light sources are positioned substantially in line within a housing to minimise interference between said light sources. However, in an alternative preferred embodiment said light sources are positioned substantially in parallel next to each other. To counteract a loss of efficiency due to (radiation) energy absorption by said light sources in the latter embodiment commonly the reflecting means will comprise a (reflective) partition wall positioned between said light sources to divide light patterns emitted by said light sources substantially.

In a particular preferred embodiment the tanning apparatus comprises multiple housings in which the filtering means and the reflecting means are incorporated, wherein each housing is adapted for accommodating at least one light source. Application of multiple housings is commonly advantageously, since each housing can be optimised to reflect and filter light emitted by the at least one light source contained by that housing to optimise the light patterns emitted by the individual housings (commonly via the filtering means), and, moreover, to optimise the overall light pattern emitted by the apparatus according to the invention in the direction of a user to allow irradiation of a user in an optimum manner. To this end, adjacent housings are preferably adapted to enclose an angle which is situated between 115° and 125°, and which angle preferably is substantially 120°. It has been found that orienting adjacent housings in such a way that they enclose an angle of between 115° and 125°, and more preferably an angle of 120°, a user can be irradiated by a relatively broad though directed emission pattern, which leads to a relatively efficient irradiation of a user.

In order to further optimise irradiation of a user during a tanning process, the orientation between the housings is preferably adjustable. In this manner the radiation pattern emitted by the apparatus according to the invention can relatively easily be adjusted and be optimised for specific circumstances. Besides adjusting the radiation pattern of the apparatus, it is commonly further advantageous to be able to adjust the orientation of adjacent housings to be able to change the orientation of said housings between an operational position, in which a substantial part of a housing lies at a distance from the adjacent housing, and a non-operational position, in which a housing is folded substantially against the adjacent housing. In this manner, after use the apparatus can easily be collapsed to a relatively compact package which facilitates storage and transport of the apparatus. For use, the adjacent housings are commonly folded out towards to operational position in which the physical contact between adjacent housings is commonly limited. To allow a practical, and hence user-friendly, handling of the apparatus according to the invention, the multiple housings of the apparatus are preferably connected by means of one or more hinges (e.g. a piano hinge). To further improve the user-friendliness of the apparatus, preferably at least one housing is provided with a handle to facilitate transport of the apparatus.

Although different kind of light sources can be applied in the apparatus according to the invention, provided that the light sources have at least a spectral band in the range of violet/blue light (400-440 nm), preferably each light source is formed by a discharge lamp, in particularly a high intensity discharge lamp. Furthermore, in accordance with an embodiment of the present invention, the light source is a Gallium, Mercury and halides gas mixture discharge lamp with peak emission in the 400-440 spectral band. Alternatively, the light source is an Excimer lamp with peak emission in the spectral range of 400-440 nm. A phosphorous coating may be needed to convert radiation generated by the Excimer light source into radiation with a spectral emission in the range of 400-440 nm. The light source could also be composed of one ore more of a group of light sources including a metal halide gas discharge lamp, an Excimer lamp, an Ion Krypton gas laser with a spectral emission in the range 400 to 440 nm, a diode and a diode matrix. The diode or diodes are selected from the group consisting of violet/blue laser diodes, and light emitting diodes (LED) with narrow spectral band emission in the spectral range of 400-440 nm. Preferably, a discharge lamp is applied which has a limited radiation in the infrared spectrum to prevent overheating of the skin (in this case a ventilator should be used for cooling the skin). In case the discharge lamp is adapted to generate a considerable quantity of infrared radiation, preferably the filtering means comprises an infrared filter.

In a preferred embodiment the reflecting means comprise at least one curved reflector to direct and converge the light in the direction of the filtering means. More preferably, the reflector is selected from the group includes of an elliptical cross-section cylindrical reflector, parabolic cross-section cylindrical reflector, and an asymmetric aspheric reflector. To improve the tolerances of the curved reflector, the curved reflector is preferably built up out of multiple facet-shaped reflective organs. In this manner the curved reflector is less sensitive for minor deviations during the production process compared to the situation wherein a smooth (instead of a facetted) parabolic cross-section cylindrical reflector is applied. To reflect light emitted by the light source(s) in an optimum manner, the light source is preferably enclosed at least partially by the curved reflector.

The reflectivity of the (at least one curved reflector of the) reflecting means is preferably relatively high, preferably at least 90%, more preferably at least 95%to optimise the light output of the tanning apparatus according to the invention and to minimise loss of efficiency due to energy absorption by the reflecting means.

The filtering means comprises preferably at least one UV barrier filter glass, such as an UVILEX^{®}-390 filter (Desag. Germany) or a Schott GG395. An UVILEX^{®}-390 filter is ideally suitable to at least substantially block ultraviolet radiation from artificial light sources, and hence to protect the human skin from harmful UV radiation.

The invention can further be illustrated by way of the following non-limitative embodiments, wherein:
Fig. 1 shows a perspective view of a tanning apparatus according to the invention in an operational state,
Fig. 2 shows a perspective view of the tanning apparatus according to figure 1 in an non-operational state,
Fig. 3 shows a front view of an optical system as used in the tanning apparatus according to figures 1 and 2, and
Fig. 4 shows a top view of an optical system as used in the tanning apparatus according to figures 1 and 2.

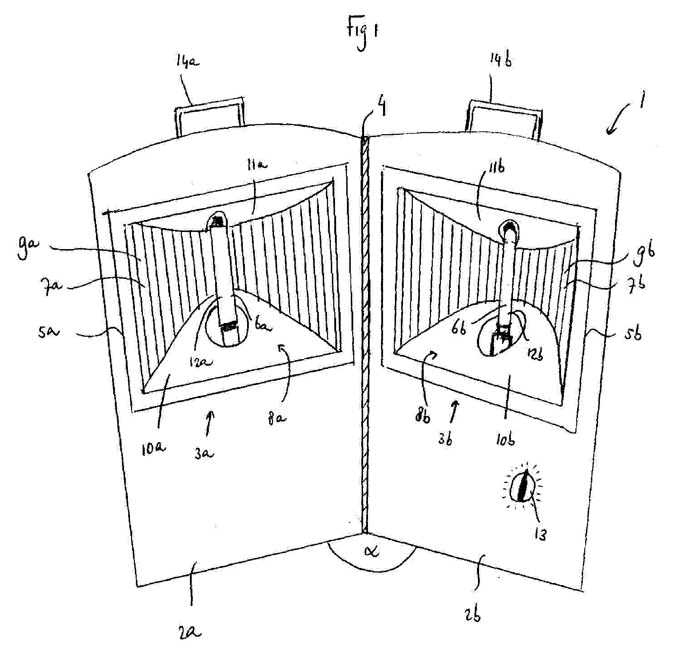

Figure 1 shows a perspective view of a tanning apparatus 1 according to the invention in an operational state. The apparatus 1 comprises a first tanning unit 2a and a second tanning unit 2b comprising two optical systems 3a, 3b respectively, wherein the tanning units 2a, 2b are mutually coupled by means of a hinge 4. Each optical system 3a, 3b comprises a housing 5a, 5b for a discharge lamp 6a, 6b, said housing 5a, 5b being defined by a reflective backing structure 7a, 7b and an UV barrier filter glass 8a, 8b. The reflective backing structure 7a, 7b comprises a parabolic cross-section facetted cylindrical reflector 9a, 9b, a reflective bottom plate 10a, 10b, and a reflective top plate 11a, 11b, both plates 10a, 10b, 11a, 11b being connected to said facetted cylindrical reflector 9a, 9b. The discharge lamps 6a, 6b used are high intensity discharge (HID) lamps (400 Watt) each lamp 6a, 6b comprising a discharge vessel 12a, 12b being filled with Mercury, and a Gallium halide salt, in particular GaI₃, which make the discharge lamp 6a, 6b suitable for emitting a considerable quantity of violet/blue light (400-440 nm) during operation. The UV barrier filter glass 8a, 8b used is a UVILEX^{®}-390 filter, which filter 8a, 8b is commercially known. The filter glass 8a, 8b is adapted to reject UV radiation (up to 400 nm) and to transmit violet/blue light emitted by the discharge lamps 6a, 6b. In this manner a person will (substantially) not be irradiated by UV radiation but rather by violet/blue radiation. Since a UV free tanning process requires an optimum, efficient light output of the tanning apparatus 1, application of multiple tanning units 2a, 2b, and a high efficiency reflector design are considered to be of major importance to enable irradiation of a tanning person (per unit of time) in an optimum manner. In the embodiment of the apparatus 1 shown, the orientation between the tanning units 2a, 2b is adjustable. During operation, the angle α enclosed by both adjacent tanning units 2a, 2b is preferably about 120° for optimum irradiating a tanning person being situated at a distance of about 25 cm from the hinge 4. This angle is dependent on the design of the reflective backing structure 7a, 7b and for other reflective backing structures possibly another angle will be preferred. By means of a timer switch 13 contained by the second tanning unit 2b the tanning time (commonly up to 30 or 60 minutes) can be adjusted by the person. Both tanning units 2a, 2b are provided with a handle 14a, 14b to facilitate transport of the tanning apparatus 1. It is noted that it is also conceivable to rotate each optical system 3a, 3b (including a discharge lamp 6a, 6b and the reflective backing structure 7a, 7b) a quarter turn to achieve a substantial horizontal instead of a substantial vertical orientation over the optical system 3a, 3b.

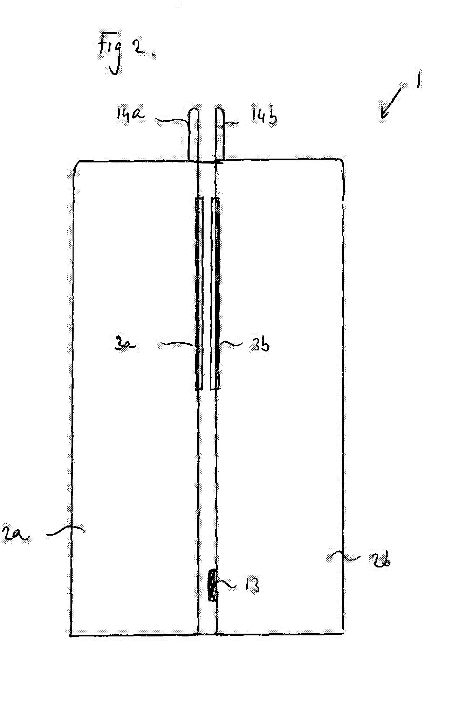

Figure 2 shows a perspective view of the tanning apparatus 1 according to figure 1 in an non-operational state. In this non-operational state the apparatus 1 is made compact by hinging the tanning units 2a, 2b towards each other. In this state the optical systems 3a, 3b of the different tanning units 2a, 2b are facing each other, and the handles 14a, 14b of both units 2a, 2b are positioned substantially in line. In the shown non-operational state of the (suitcase-like) tanning apparatus 1 both transport and storage of the apparatus 1 can be facilitated.

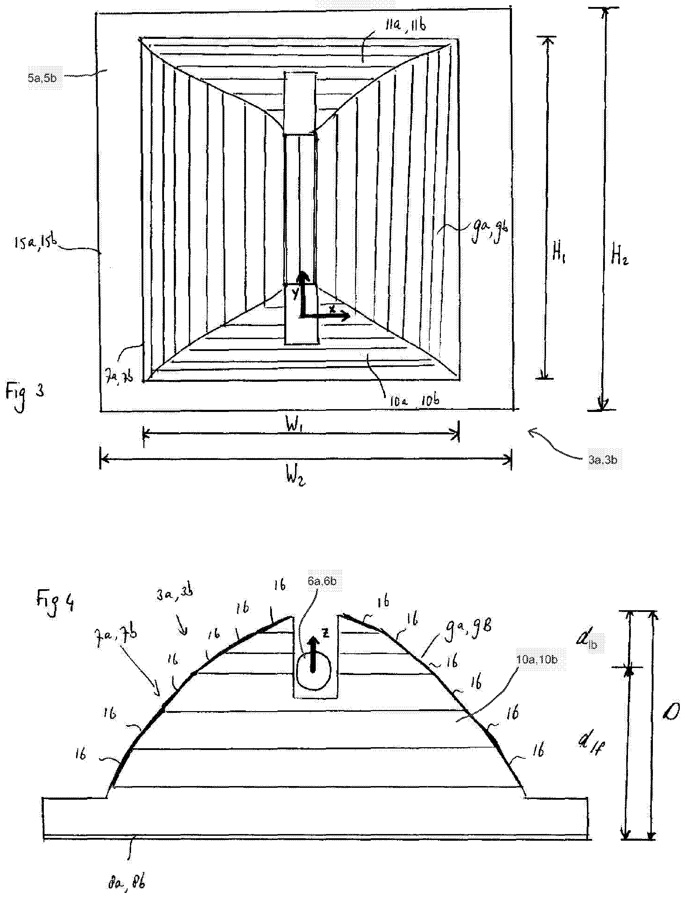

Figure 3 shows a front view of an optical system 3a, 3b as used in the tanning apparatus 1 according to figures 1 and 2. As shown in this figure 3 the discharge lamp 6a, 6b has been positioned centrally within the housing 5a, 5b defined by the reflective backing structure 7a, 7b, and the UV barrier filter glass 8a, 8b (not shown in Figure 3). In this context it is remarked that a tolerance of -2 or +2 mm in both the X-direction and the Y-direction (see arrows) would be allowable to maintain a satisfying light output of the optical system 3a, 3b shown. In the embodiment shown the width W₁ of the reflective backing structure 7a, 7b is about 16,9 cm and the height H₁ of the reflective backing structure 7a, 7b is about 18,5 cm. A border 15a, 15b of the optical system 3a,3b has a width W₂ of about 22,2 cm and a height H₂ of about 21,5 cm. It may be clear that these dimensions may be adapted by a person skilled in the art dependent on situational circumstances. It is noted that the design of the reflective backing structure 7a, 7b is dependent on the geometry of the discharge lamp 6a, 6b applied; changing the type of discharge lamp 6a, 6b will commonly also require modifications of the reflective backing structure 7a, 7b to maintain an optimum reflectance of the backing structure 7a, 7b.

Figure 4 shows a top view of an optical system 3a, 3b as used in the tanning apparatus 1 according to figures 1 and 2. In this figure it is shown that the parabolic cross-section cylindrical reflector 9a, 9b of the reflective backing structure 7a, 7b is constructed out of multiple facets 16 to facilitate the production process of the cylindrical reflector 9a, 9b on one side and to make the design of the reflector 9a, 9b somewhat less critical. In this embodiment the distance d_{1b} of the discharge lamp 6a, 6b to the back side of the optical system 3a, 3b is about 2,3 cm, and the distance d_{1f} of the discharge lamp 6a, 6b to the front side of the optical system 3a, 3b is about 7 cm. The total depth D of the optical system 3a, 3b is about 9,3 cm in this embodiment. In this context it is noted that the position of the discharge lamp 6a, 6b (see arrow) could slightly be changed in the Z-direction (approximately from -1 mm to +2 mm) without (notably) affecting the efficiency of the optical system 3a, 3b.

It should be noted that the above-mentioned embodiments illustrate rather than limit the invention, and that those skilled in the art will be able to design many alternative embodiments without departing from the scope of the appended claims. In the claims, any reference signs placed between parentheses shall not be construed as limiting the claim. Use of the verb "comprise" and its conjugations does not exclude the presence of elements or steps other than those stated in a claim. The article "a" or "an" preceding an element does not exclude the presence of a plurality of such elements. The mere fact that certain measures are recited in mutually different dependent claims does not indicate that a combination of these measures cannot be used to advantage.

## Claims

1. Tanning apparatus, comprising:
- multiple light sources (6a, 6b) with a spectral emittance concentrated in at least one specific narrow spectral band, wherein one spectral band is in the range of 400-440 nm,
- filtering means (8a, 8b) for at least substantially rejecting ultraviolet radiation emitted by said light sources, and
- reflecting means (9a, 9b) for directing light emitted by said light sources towards said filtering means.

2. Tanning apparatus according claim 1, **characterized in that** the tanning apparatus comprises at least one housing (5a, 5b) for at least one light source, in which housing the filtering means and the reflecting means are incorporated.

3. Tanning apparatus according to claim 2, **characterized in that** the housing is adapted for containing multiple light sources.

4. Tanning apparatus according to claim 2 or 3, **characterized in that** the tanning apparatus comprises multiple housings in which the filtering means and the reflecting means are incorporated, wherein each housing is adapted for accommodating at least one light source.

5. Tanning apparatus according to claim 4, **characterized in that** adjacent housings are adapted to enclose an angle which is situated between 115° and 125°, and which angle preferably is substantially 120°.

6. Tanning apparatus according to claim 4 or 5, **characterized in that** the orientation between the housings is adjustable.

7. Tanning apparatus according to claim 6, **characterized in that** the mutual orientation between adjacent housings can be changed between an operational position, in which a substantial part of a housing lies at a distance from the adjacent housing, and a non-operational position, in which a housing is folded substantially against the adjacent housing.

8. Tanning apparatus according to one of claims 2-7, comprises that at least one housing is provided with a handle (14a, 14b).

9. Tanning apparatus according to one of the foregoing claims, **characterized in that** each light source is formed by a discharge lamp, in particularly a high intensity discharge lamp.

10. Tanning apparatus according to one of the foregoing claims, **characterized in that** the reflecting means comprise at least one curved reflector.

11. Tanning apparatus according to claim 10, **characterized in that** the curved reflector is defined by multiple facet-shaped reflective organs (16).

12. Tanning apparatus according to claim 10 or 11, **characterized in that** the curved reflector partially encloses at least one light source.

13. Tanning apparatus according to one of the foregoing claims, **characterized in that** the reflectivity of the reflecting means is at least 90%, more preferably at least 95%.
